# EUROPÄISCHE PATENTSCHRIFT

(11) **EP 1 660 097 B1**
(45) Veröffentlichungstag und Bekanntmachung des Hinweises auf die Patenterteilung: **04.02.2009**
(21) Anmeldenummer: 04764814.2
(22) Anmeldetag: 03.09.2004
(51) Int. Cl.: A61K 31/685, A61K 31/683, A61K 31/661

(54) **PHYSIOLOGISCH AKTIVE ZUSAMMENSETZUNG AUF PHOSPHATIDYLSERIN-BASIS**
PHYSIOLOGICALLY ACTIVE COMPOSITION BASED ON PHOSPHATIDYLSERINE
COMPOSITION PHYSIOLOGIQUEMENT ACTIVE A BASE DE PHOSPHATIDYLSERINE

(30) Priorität: 04.09.2003 DE 10340740
(43) Veröffentlichungstag der Anmeldung: 31.05.2006
(73) Patentinhaber: Cargill Texturizing Solutions Deutschland GmbH & Co KG, 20539 Hamburg (DE)
(72) Erfinder: PURPURA, Martin, 53227 Bonn (DE); JÄGER, Ralf, Milwaukee, WI 53202 (US); RABELER, Roland, 85356 Freising (DE); HOPPE, Hans-Ullrich, 85416 Langenbach (DE)
(74) Vertreter: Böhm, Brigitte
(86) Internationale Anmeldenummer: PCT/EP2004/009862
(87) Internationale Veröffentlichungsnummer: WO 2005/023271

(56) Entgegenhaltungen:
- EP-A- 0 342 795
- EP-A- 0 711 559
- US-A- 4 221 784
- US-A- 6 117 853
- ZANOTTI A ET AL: "CHRONIC PHOSPHATIDYLSERINE TREATMENT IMPROVES SPATIAL MEMORY AND PASSIVE AVOIDANCE IN AGED RATS" PSYCHOPHARMACOLOGY, SPRINGER VERLAG, BERLIN, DE, Bd. 99, Nr. 3, 1989, Seiten 316-321, XP000603396 ISSN: 0033-3158

## Beschreibung

Gegenstand der vorliegenden Erfindung ist eine physiologisch aktive Zusammensetzung auf Phosphatidylserin-Basis gemäß Anspruch 1.

Phosphatidylserin (PS) ist ein natürliches Phospholipid mit typischerweise amphiphilen Eigenschaften. Gemeinsam mit anderen Vertretern der Phospholipide ist es am Aufbau von biologischen Membranen beteiligt. Die entsprechenden Lysoformen tragen in C₁-oder in C₂-Stellung des Glycerin-Restes keine Fettsäure, sondern eine einfache Hydroxygruppe.

Aufgrund ihrer chemischen Eigenschaften sind Phospholipide allgemein und Phosphatidylserin sowie seine Lyso-Variante im Besonderen am Aufbau des Gehirns, am allgemeinen Stoffwechselgeschehen und bei der Weiterleitung und Verarbeitung von neuronalen Informationen beteiligt.

Hinlänglich bekannt ist auch der positive Einfluss von Phosphatidylserin auf die Gehirnfunktion, wenn es von extern zugeführt wird.

So ist bspw. mit dem US-Patent 5,900,409 eine Zusammensetzung zur Stärkung der Gehirnfunktion geschützt, die Phosphatidylserin enthält, welches durch eine Transphosphatidylierung mit Phospholipase D hergestellt wurde. Ebenso wird in Zanotti, A. et al., Psychopharmacology, Bd. 99, Nr. 3, 1989, Seiten 316-321 beschrieben, dass Phosphatidylserine das Erinnerungsvermögen verbessert. US 6,117,853 beschreibt eine entsprechende Zusammensetzung, die als Hauptwirkstoff Lyso-Phosphatidylserin enthält.

Wurde Phosphatidylserin in der Vergangenheit ausschließlich aus Gehirnsubstanz bspw. von Rindern und Schweinen gewonnen, so hat sich im letzten Jahrzehnt die Herstellung von PS in kommerziellen Mengen durch eine enzymatische Transphosphatidylierung unter Beteiligung von Phospholipase D durchgesetzt.

Nicht nur Phosphatidylserin bzw. Lyso-Phosphatidylserin können positiven Einfluss auf die Gehirnfunktion nehmen, sondern auch zahlreiche andere Verbindungen, wie bspw. Omega-3- und Omega-6-Fettsäuren, die ebenfalls essentielle Bestandteile neuronaler Gewebe, bspw. im Gehirn, darstellen.

Aus EP-A 0 342 795 ist bspw. ebenfalls eine Zusammensetzung zur Stärkung der Gehirnfunktion bekannt, die Docosahexaensäure (DHA), also eine typische Omega-3-Fettsäure, enthält.

Omega-3- und Omega-6-Fettsäuren werden aber nicht nur als Monopräparate verwendet, sondern aufgrund ihrer hauptsächlichen Quelle, den Fischölen, meist in Kombinationspräparaten, wobei sie dann in verkapselter Form verabreicht werden.

Fischöle und deren Hauptinhaltsstoffe, die Fettsäuren, werden in vielfältiger Weise eingesetzt und sollen positive Wirkungen bei entzündlichen Erkrankungen, bspw. von Gelenken, bei der Verbesserung von sportlichen Leistungen, aber auch zur Senkung des Serumcholesterinspiegels entfalten. Verwiesen sei in diesem Zusammenhang beispielhaft auf GB 2209936, in der eine Fischölzubereitung zur Behandlung von arthritischen Symptomen gelehrt wird, oder aber auf WO 93/21912, die ebenfalls zur Behandlung von entzündlichen Erkrankungen die Verwendung einer Omega-3-Fettsäure-haltigen Emulsion empfiehlt. Ebenfalls zur Behandlung von Arthritis wird gemäß US 5,843,919 eine Kombination aus Omega-3-Fettsäuren und Glucosamin eingesetzt. Die Behandlung von Gelenkserkrankungen, Gelenksschmerzen und Steifheitserscheinungen von Gelenken mit Hilfe von Hagebuttekonzentraten und Fischölen wird durch US 6,485,752 geschützt. Gemäß EP-A 1155620 wird eine Kombination unterschiedlicher Vitamine mit Mineralstoffen und mindestens 40 Gew.-% eines Fischöl-Granulats in Form eines Nahrungsergänzungsmittels beansprucht. EP-A 1004303 schlägt zur Erniedrigung von Risikofaktoren während sportlicher Übungen eine Zusammensetzung vor, die hoch-ungesättigte Omega-3-Fettsäuren enthält.

Im allgemeinen besteht bei oral verabreichten Zusammensetzungen das Problem, dass die Formulierungen und insbesondere deren physiologisch aktive Inhaltsstoffe die Magen-Darm-Passage so überstehen, dass sie in aktiver Form den für sie bestimmten Wirkort erreichen. Dies setzt voraus, dass sie die unterschiedlichen pH-Milieus des Magen-Darm-Traktes sowie die darin ablaufenden enzymatischen Prozesse in einer Weise überstehen, dass ihre physiologische Wirksamkeit nicht negativ verändert wird.

Zu diesem Zweck müssen die meisten Wirkstoffe in hoch-dosierter Form oral zugeführt oder so formuliert werden, dass eine ausreichende Menge der unveränderten Verbindung an den Wirkort gelangt. Bei der oralen Verabreichung von bspw. Phosphatidylserin führen eine Reihe von Prozessen zu einer massiven Reduktion der finalen Konzentration am Wirkort. So wird ein großer Anteil des im Magen-Darm-Trakt aufgenommenen Phosphatidylserins schon in den Darmzellen irreversibel in andere Phospholipide umgewandelt. Gleichzeitig führen gezielte enzymatische Abbauprozesse zu einer weiteren Reduktion der effektiven Menge an Phosphatidylserin.

Aufgrund dieses bekannten Nachteils des Standes der Technik hat sich für die vorliegende Erfindung die Aufgabe gestellt, eine physiologisch aktive Zusammensetzung auf Phosphatidylserin-Basis bereitzustellen, mit der insbesondere bei oraler Verabreichung ausreichend hohe Wirkstoffmengen von Phosphatidylserin oder deren Lysoform an den unterschiedlichen möglichen Wirkorten im Körper erreicht werden, ohne dass komplexe Formulierungen oder spezielle Darreichungsformen appliziert werden müssen.

Gelöst wurde diese Aufgabe mit einer entsprechenden Zusammensetzung, die als physiologisch aktive Bestandteile die Komponenten
a) Phosphatidylserin und/oder Lyso-Phosphatidylserin (jeweils "PS") und
b) mindestens ein von Komponente a) verschiedenes (Lyso-)Phospholipid ausgewählt aus Phosphatidylcholin, Phosphatidylethanolamin und Phosphatidylinosit und
c) eine von Komponente a) verschiedene Serin-Quelle, wie z.B. L-Serin und/oder O-Phospho-L-Serin,
enthält, wobei die molaren Verhältnisse a : b und a: c 1,0 : 1,0 bis 100 betragen.

Überraschend wurde mit dieser Zusammensetzung gefunden, dass die eingesetzten Mengen an Komponente b) und c) unter physiologischen Bedingungen geeignet sind, die Konzentration von Phosphatidylserin am Wirkort (insbesondere Gehirn) signifikant zu erhöhen. Dabei werden zum einen die Komponenten b) und c) im Körper zu Phosphatidylserin umgesetzt, zum anderen bewirkt ihr Vorhandensein einen Ausgleich des PS-Verlustes durch Abbau und Umbau, aber auch einen verbesserten PS-Transport zum Wirkort. Dies geschieht in einem Ausmaß, dass die beiden Komponenten b) und c) nicht nur die Mengen an üblicherweise verabreichtem und/oder unter physiologischen Bedingungen katabolisiertem Phosphatidylserin auszugleichen vermögen, sondern gemeinsam mit den während der Körperpassage unverändert wirksam bleibenden Phosphatidylserin-Mengen zu einer Gesamt-PS-Menge am jeweiligen Wirkort führen, die eine drastische Reduzierung an eingesetztem Phosphatidylserin ermöglicht. Dieser überraschende Effekt hat nicht nur wirtschaftliche Konsequenzen, sondern es könnte auch die gezielte Vorausberechnung der am Hauptwirkort, dem Gehirn, ankommenden PS-Menge möglich werden, was insbesondere im Hinblick auf die positive Beeinflussung von Distress-Erscheinungen bei körperlicher und/oder geistiger Belastung von Bedeutung ist.

Als besonders effektiv hat sich eine erfindungsgemäße Zusammensetzung gezeigt, deren molare Verhältnisse a : b und a : c 1,0 : 1,0 bis 100 betragen.

Im Rahmen der vorliegenden Erfindung hat sich aber auch eine Variante der beanspruchten Zusammensetzung als besonders geeignet erwiesen, bei der unter in-vivo-Bedingungen aus den Komponenten b) und c) PS gebildet werden kann, und zwar vorzugsweise in Mengen, die 10 bis 99 Gew.-% des Anteils der Komponente a), also Phosphatidylserin und/oder Lyso-Phosphatidylserin, an der Gesamtformulierung ersetzt.

In diesem Zusammenhang ist auch eine erfindungsgemäße Zusammensetzung als bevorzugt anzusehen, die 0,1 bis 20 Ges.-%, bezogen auf die Gesamtformulierung, der Komponente a), enthält. Diese Variante verdeutlicht das mit der erfindungsgemäßen Zusammensetzung verbundene Potential zur Senkung extern verabreichter PS-Mengen.

Da der Hauptvorteil der beanspruchten Zusammensetzung also darin besteht, dass Phosphatidylserin und/oder Lyso-Phosphatidylserin-Anteile durch die kombinierte Verabreichung mit den Komponenten b) und c) drastisch gesenkt werden können, berücksichtigt die vorliegende Erfindung auch eine Zusammensetzung, in der die Komponente b) in Anteilen zwischen 15 und 65 Ges.-% und die Serin-Komponente c) in Anteilen zwischen 0,1 und 5,0 Gew.-%, wieder jeweils bezogen auf die Gesamtformulierung, vorliegen.

Typische Vertreter der Komponente b) sind das Phosphatidylcholin (PC), das Phosphatidylethanolamin (PE) und das Phosphatidylinosit (PI), die natürlich auch als Lyso-Varianten in der beanspruchten Zusammensetzung eingesetzt werden können; aber auch andere geeignete (Lyso-)Phospholipide kommen als Komponente b) in Frage.

Bezüglich der weiteren Hauptkomponente c), also der von PS verschiedenen Serin-Quelle, ist die vorliegende Zusammensetzung im Rahmen der beanspruchten Voraussetzungen keinerlei Beschränkung unterworfen. Es muss lediglich gewährleistet sein, dass unter physiologischen Bedingungen Serin tatsächlich für den gewünschten Zweck verfügbar ist.

Neben den drei erfindungswesentlichen Komponenten a, b und c kann die beanspruchte Zusammensetzung auch noch weitere physiologisch aktive Bestandteile enthalten, wobei vorzugsweise zusätzlich Omega-3-Fettsäuren in Frage kommen. In diesem Zusammenhang sieht die vorliegende Erfindung einen Anteil vor, der zwischen 30 und 80 Gew.-%, bezogen auf die Gesamtformulierung, liegt. Damit ist die Möglichkeit geschaffen, die physiologische PS-Wirkung einerseits zu unterstützen oder aber synergistisch zu verstärken. Vorteilhaft dabei ist aber auch, dass diese zusätzliche Komponente nicht nur als Wirkstoff dient, sondern gleichermaßen gut auch als Matrix für die drei Hauptkomponenten.

Besonders vorteilhaft hat sich der Einsatz von Docosahexaensäure (DHA), α-Linolensäure (ALA) und Eicosapentaensäure (EPA), insbesondere in Form von Mono-, Di- und/oder Triglyceriden und Monoestern oder aber in Form von beliebigen Mischungen als typische Vertreter der zusätzlichen Fettsäure-Komponente erwiesen.

Wie bereits erwähnt, kann die beanspruchte Zusammensetzung neben den Hauptinhaltsstoffen a) bis c) und den Omega-3-Fettsäuren auch noch weitere Komponenten enthalten, die entweder selbst eine physiologische Wirkung entfalten oder lediglich als Formulierungshilfsmittel eingesetzt werden. Bevorzugt sind dabei als weitere Bestandteile und insbesondere in Form zusätzlicher physiologisch aktiver Bestandteile solche mit durchblutungsfördernder Wirkung, wie z.B. Ginko biloba, oder solche mit neuroprotektiver und/oder antioxidativer Wirkung, wie z.B. Vitamin A, Vitamin C, Vitamin E, Polyphenole, beta-Carotin, Selen und α-Liponsäure, aber auch solche Bestandteile mit Gehirnstoffwechsel-stimulierender Wirkung, wie bspw. Komplexvitamine (B 1, B 6, B 12, Folsäure), Omega-6-Fettsäuren, Kreatin, Koffein und Kohlenhydrate (Glucose). Vorzugsweise können aber auch Substanzen enthalten sein, die die Synthese oder Freisetzung von Neurotransmittern positiv beeinflussen, wie z.B. Cholin sowie deren (an-)organische Salze oder Acetyl-Donatoren, wie z.B. Acetylcholin, und/oder Substanzen, die die Bioverfügbarkeit, die Verteilung und den Metabolismus von Phospholipiden positiv beeinflussen, wie z.B. PPAR-Agonisten (also Peroxisome-Proliferator-Activated-Rezeptor-Agonisten, die meist auf Thiazolidindion-Basis bei der Diabetes Typ II-Behandlung insulinsensitivierend und lipidsenkend wirken), Retinsäure-Rezeptor-Agonisten und Blut-Cholesterin-Senker allgemein. Schließlich kommen als weitere Bestandteile Formulierungshilsmittel in Frage, wie z.B. Füllstoffe, Trennmittel, Aromastoffe und Farbstoffe, wobei sämtliche genannten weiteren Bestandteile natürlich auch in beliebiger Mischung in der beanspruchten Zusammensetzung enthalten sein können.

Neben den Komponenten der erfindungsgemäßen Zusammensetzung umfasst die vorliegende Erfindung auch deren Verabreichungsform, wobei flüssige Formulierungen und insbesondere solche in Kapselform oder als Pulver und insbesondere als Tablette oder Dragee als bevorzugt anzusehen sind. Werden Kapseln eingesetzt, enthalten diese als Trägermatrix meist Fischöle; Pulver bestehen oder enthalten microverkapselte Fischöle, bei denen es sich auch um Omega-3-Fettsäuren handeln kann.

Die vorliegende Erfindung berücksichtigt auch die Verwendung der erfindungsgemäßen Zusammensetzung zur Herstellung eines Mittels zur Verbesserung und Stärkung der Gehirn- und Gedächtnisfunktion, sowie deren weitere bevorzugte Verwendung als Nahrungsergänzungsmittel (Dietary Supplements), Funktionsnahrungsmittel (Functional Food) oder als Spezialernährung.

Ein weiterer Aspekt der erfindungsgemäßen Verwendung steht im Zusammenhang mit körperlichem und geistigem Distress, wie er insbesondere im Rahmen sportlicher Aktivitäten auftreten kann, und dem mit der erfindungsgemäßen Zusammensetzung vorgebeugt werden kann oder dessen Symptome unter Supplementation drastisch reduziert werden.

Zusammenfassend stellt die erfindungsgemäße, physiologisch aktive Zusammensetzung ein neues Mittel dar, mit dem es möglich ist, die Bioverfügbarkeit, die Verteilung und den Metabolismus von Phospholipiden im Körper positiv zu beeinflussen. Das Phosphatidylserin oder dessen Lyso-Form kann dabei zum überwiegenden Teil mittels physiologischer in-vivo-Prozesse aus den angebotenen, physiologischen Vorläufersubstanzen b) und c) hergestellt werden, wodurch die Menge an tatsächlich oral zugeführtem PS (Komponente a)) deutlich gesenkt werden kann. Die oral zugeführte (Lyso-)Phosphatidyl-Menge wird außerdem in ihrer Wirkung am Zielort dadurch unterstützt, dass in-vivo aus den Komponenten b) und c) zusätzliche PS-Mengen gebildet werden, die gemeinsam mit dem verabreichten (Lyso-)Phosphatidylserin (Komponente a)) und zusätzlichen Komponenten, wie z.B. Omega-3-Fettsäuren, am Hauptwirkort, nämlich dem Gehirn, zu einer verbesserten Funktion und Leistungsfähigkeit führen.

Die nachfolgenden Beispiele verdeutlichen die Vorteile der vorliegenden Erfindung.

### Beispiele

### Beispiel 1:

10 g Phosphatidylserin (Leci PS^{®} 90PN von Degussa Food Ingredients GmbH) wurden als Komponente a) in 180 g der Omega-3-Fettsäure Docosahexaensäure (Marinol^{™} D-50 TG von Loders Crooklan) gegeben und in einem Labormischer so lange gerührt, bis eine homogene Mischung resultierte. Anschließend wurden als Komponente b) 180 g Phosphatidylcholin (Epikuron^{™} 135F von Degussa Food Ingredients GmbH) hinzugegeben und solange gerührt, bis eine homogene Lösung erhalten wurde. Zum Schluss wurden 28 g der Aminosäure L-Serin (Komponente c) von Degussa Fine Chemicals) hinzugegeben. Die so erhaltene Dispersion wurde im Anschluss in Weichgelatinekapseln mit einem Füllgewicht von 500 mg und einem Gesamtgewicht von 700 mg eingearbeitet.
Pro Kapsel waren als physiologisch aktive Zusammensetzung (in Gewichtsprozent) enthalten:
1 % Phosphatidylserin
18 % Docosahexaensäure (Omega-3 Fettsäure)
18 % Phosphatidylcholin (Phospholipid)
2,8 % L-Serin

### Molare Verhältnisse:

Phosphatidylserin : Phosphatidylcholin : L-Serin =1 : 19 : 21

### Beispiel 2:

50 g Phosphatidylserin (Leci PS^{®} 90PN von Degussa Food Ingredients GmbH) wurden als Komponente a) in 140 g Phosphatidylcholin (Epikuron^{™} 135F von Degussa Food Ingredients GmbH) als Komponente b) gegeben und in einem Labormischer so lange gerührt, bis eine homogene Mischung resultierte. Anschließend wurden 28 g der Aminosäure L-Serin (Komponente c) von Degussa Fine Chemicals) hinzugegeben. Die so erhaltene Dispersion wurde im Anschluss in Weichgelatinekapseln mit einem Füllgewicht von 300 mg und einem Gesamtgewicht von 450 mg eingearbeitet.
Pro Kapsel waren als physiologisch aktive Zusammensetzung (in Gewichtsprozent) enthalten:
5 % Phosphatidylserin
14 % Phosphatidylcholin (Phospholipid)
2,8 % L-Serin

### Molare Verhältnisse:

Phosphatidylserin : Phosphatidylcholin : L-Serin =1 : 3 : 4

### Beispiel 3:

10 g Phosphatidylserin (Leci PS^{®} 90PN von Degussa Food Ingredients GmbH) wurden als Komponente a) in 160 g der Omega-3-Fettsäure Docosahexaensäure (Marinol^{™} D-50 TG von Loders Crooklan) gegeben und in einem Labormischer so lange gerührt, bis eine homogene Mischung resultierte. Anschließend wurden 180 g Phosphatidylcholin (Epikuron^{™} 135F von Degussa Food Ingredients GmbH) als Komponente b) hinzugegeben und so lange gerührt, bis eine homogene Lösung erhalten wurde. Diese Mischung wurde kurzeitig auf 55 °C erwärmt und mit 50 g Bienenwachs als Formulierungs-Hilfsmittel versetzt. Zum Schluss wurden 28 g der Aminosäure L-Serin (von Degussa Fine Chemicals) als Komponente c) hinzugegeben. Die so erhaltene Dispersion wurde im Anschluss in Weichgelatinekapseln mit einem Füllgewicht von 500 mg und einem Gesamtgewicht von 700 mg eingearbeitet.
Pro Kapsel waren als physiologisch aktive Zusammensetzung (in Gewichtsprozent) enthalten:
1 % Phosphatidylserin
16 % Docosahexaensäure (Omega-3 Fettsäure)
18 % Phosphatidylcholin (Phospholipid)
2,8 % L-Serin

### Molare Verhältnisse:

Phosphatidylserin: Phosphatidylcholin: L-Serin = 1 : 19 : 21

### Beispiel 4:

10 g Phosphatidylserin (Leci PS^{®} 90PN von Degussa Food Ingredients GmbH), 70 g Docosahexaensäure (Marinol^{™} DHA Powder von Loders Crooklan) als zusätzliche Omega-3-Fettsäure, 180 g Phosphatidylcholin (Epikuron^{™} 130P von Degussa Food Ingredients GmbH) als Komponente b) und 28 g L-Serin (Komponente c) von Degussa Fine Chemicals) wurden als Pulvermischung in Hartgelatinekapseln eingearbeitet. Diese wiesen ein Füllgewicht von 500 mg und ein Gesamtgewicht von 700 mg auf
Pro Kapsel waren als physiologisch aktive Zusammensetzung (in Gewichtsprozent) enthalten:
1 % Phosphatidylserin
7 % Docosahexaensäure (Omega-3 Fettsäure)
18 % Phosphatidylcholin (Phospholipid)
2,8 % L-Serin

### Molare Verhältnisse:

Phosphatidylserin: Phosphatidylcholin: L-Serin =1 : 19 : 21

## Patentansprüche

1. Physiologisch aktive Zusammensetzung, enthaltend als physiologisch aktive Bestandteile die Komponenten
a) Phosphatidylserin und/oder Lyso-Phosphatidylserin (jeweils "PS") und
b) mindestens ein von Komponente a) verschiedenes (Lyso-)Phospholipid, ausgewählt aus Phosphatidylcholin, Phosphatidylethanolamin und Phosphatidylinosit, und
c) eine von Komponente a) verschiedene Serin-Quelle, ausgewählt aus L-Serin und/oder O-Phospho-L-Serin,
wobei die molaren Verhältnisse a : b und a : c jeweils 1,0 : 1,0 bis 100 betragen.

2. Zusammensetzung nach Anspruch 1, **dadurch gekennzeichnet, dass** unter in-vivo-Bedingungen aus den Komponenten b) und c) PS gebildet werden kann und vorzugsweise in Mengen, die 10 bis 99 Gew.-% des Anteils der Komponente a) an der Gesamtformulierung ersetzt.

3. Zusammensetzung nach Anspruch 1 oder 2, **dadurch gekennzeichnet, dass** sie 0,1 bis 20 Gew.-%, bezogen auf die Gesamtformulierung, der Komponente a) enthält.

4. Zusammensetzung nach einem der Ansprüche 1 bis 3, **dadurch gekennzeichnet, dass** die Komponente b) in Anteilen zwischen 15 und 65 Gew.-% und die Serin-Komponente c) in Anteilen zwischen 0,1 und 5,0 Gew.-%, jeweils bezogen auf die Gesamtformulierung, vorliegen.

5. Zusammensetzung nach einem der Ansprüche 1 bis 4, **dadurch gekennzeichnet, dass** sie zusätzlich eine Omega-3-Fettsäure enthält, insbesondere in Mengen zwischen 30 und 80 Gew.-%, bezogen auf die Gesamtformulierung.

6. Zusammensetzung nach Anspruch 5, **dadurch gekennzeichnet, dass** sie als zusätzliche Fettsäure-Komponente Docosahexaensäure (DHA), α-Linolensäure (ALA) und Eicosapentaensäure (EPA), vorzugsweise in Form von Mono-, Di- und/oder Triglyceriden und Monoestern, oder deren Mischungen enthält.

7. Zusammensetzung nach einem der Ansprüche 1 bis 5, **dadurch gekennzeichnet, dass** sie weitere Bestandteile, insbesondere in Form zusätzlicher physiologisch aktiver Bestandteile mit durchblutungsfördernder Wirkung, wie z. B. Gingko biloba, mit neuroprotektiver und/oder antioxidativer Wirkung, wie z. B. Vitamin A, Vitamin C, Vitamin E, Polyphenole, β-Carotin, Selen, α-Liponsäure, mit Gehirnstoffwechsel-stimulierender Wirkung, wie z. B. Komplex-Vitamine (B₁, B₆, B₁₂, Folsäure), Omega-6-Fettsäuren, Kreatin, Koffein und Kohlenhydrate (Glucose), und/oder Substanzen enthält, die die Synthese oder Freisetzung von Neurotransmittern positiv beeinflussen, wie z. B. Cholin sowie deren (an-)organische Salze, Acetyl-Donatoren, wie z. B. Acetylcholin, und/oder Substanzen, die die Bioverfügbarkeit, die Verteilung und den Metabolismus von Phospholipiden positiv beeinflussen, wie z.B. PPAR-Agonisten, Retinsäure-Rezeptor-Agonisten und Blut-Cholesterin-Senker, und/oder weitere Bestandteile in Form von Formulierungshilfsmitteln, wie Füllstoffe, Trennmittel, Aromastoffe, Farbstoffe.

8. Zusammensetzung nach einem der Ansprüche 1 bis 7, **dadurch gekennzeichnet, dass** sie als flüssige Formulierung, bevorzugt in Kapselform, oder als Pulver, bevorzugt als Tablette oder Dragee, vorliegt.

9. Verwendung der Zusammensetzung nach einem der Ansprüche 1 bis 8, zur Herstellung eines Mittels zur Verbesserung und Stärkung der Gehirn- und Gedächtnisfunktion.

10. Verwendung der Zusammensetzung nach einem der Ansprüche 1 bis 8 als Nahrungsergänzungsmittel (Dietary Supplement), Funktionsnahrungsmittel (Functional Food) oder als Spezialernährung.

11. Verwendung nach einem der Ansprüche 9 oder 10 im Zusammenhang mit körperlichem und geistigem Distress, insbesondere im Rahmen sportlicher Aktivitäten.

## Claims

1. Physiologically active composition containing, as physiologically active constituents, the components
a) phosphatidylserine and/or lysophosphatidylserine (in each case "PS") and
b) at least one (lyso)phospholipid different from component a), selected from phosphatidylcholine, phosphatidylethanolamine and phosphatidylinositol, and
c) a serine source different from component a), selected from L-serine and/or O-phospho-L-serine,
the molar ratios a:b and a:c in each case being 1.0:1.0 to 100.

2. Composition according to claim 1, **characterized in that**, under in vivo conditions, PS can be formed from the components b) and c) and preferably in amounts replacing 10 to 99% by weight of the fraction of component a) in the total formulation.

3. Composition according to claim 1 or 2, **characterized in that** it contains 0.1 to 20% by weight, based on the total formulation, of

4. Composition according to one of claims 1 to 3, **characterized in that** component b) is present in fractions between 15 and 65% by weight, and the serine component c) is present in fractions between 0.1 and 5.0% by weight, in each case based on the total formulation.

5. Composition according to one of claims 1 to 4, **characterized in that** it additionally contains an omega-3 fatty acid, in particular in amounts between 30 and 80% by weight, based on the total formulation.

6. Composition according to claim 5, **characterized in that**, as additional fatty acid component, it contains docosahexaenoic acid (DHA), α-linolenic acid (ALA) and eicosapentaenoic acid (EPA), preferably in the form of mono-, di- and/or triglycerides and monoesters, or mixtures thereof.

7. Composition according to one of claims 1 to 5, **characterized in that** it contains further constituents, in particular in the form of additional physiologically active constituents having circulation-promoting activity, e.g. Gingko biloba, having neuroprotective and/or antioxidant activity, e.g. vitamin A, vitamin C, vitamin E, polyphenols, β-carotene, selenium, α-lipoic acid, having activity stimulating brain metabolism, e.g. complex vitamins (B₁, B₆, B₁₂, folic acid), omega-6 fatty acids, creatine, caffeine and carbohydrates (glucose) and/or contains substances which beneficially affect the synthesis or release of neurotransmitters, e.g. choline and also (in)organic salts thereof, acetyl donators, e.g. acetylcholine, and/or substances which beneficially affect the bioavailability, distribution and metabolism of phospholipids, e.g. PPAR agonists, retinoic acid receptor agonists and blood cholesterol-decreasing compounds, and/or further constituents in the form of formulation aids, such as fillers, release agents, flavorings and colors.

8. Composition according to one of claims 1 to 7, **characterized in that** it is present as liquid formulation, preferably in capsule form, or as powder, preferably as tablet or dragee.

9. Use of the composition according to one of claims 1 to 8 for producing a means for improving and strengthening the brain and memory function.

10. Use of the composition according to one of claims 1 to 8 as dietary supplement, functional food or special nutrient.

11. Use according to either claim 9 or 10 in association with physical and mental distress, in particular in the context of sporting activities.

## Revendications

1. Composition physiologiquement active contenant comme composants physiologiquement actifs, les composants
a) phosphatidylsérine et/ou lyso-phosphatidylsérine (chacun "PS") et
b) au moins un (lyso)phospholipide différent des composants a), choisi dans le groupe comprenant la phosphatidylcholine, la phosphatidyléthanolamine et le phosphatidylinosite, et
c) une source de sérine différente des composants a), choisie parmi la L-sérine et/ou la O-phospho-L-sérine,
les rapports molaires a:b et a:c étant chacun de 1,0 : 1,0 à 100.

2. Composition selon la revendication 1, **caractérisée en ce que**, dans des conditions in vivo, une PS peut être formée à partir des composants b) et c) et de préférence, en quantités qui remplacent 10 à 99 % en poids de la proportion des composants a) dans la formulation totale.

3. Composition selon la revendication 1 ou 2, **caractérisée en ce qu'**elle contient 0,1 à 20 % en poids par rapport à la formulation totale des composants a).

4. Composition selon l'une des revendications 1 à 3, **caractérisée en ce que** les composants b) se présentent en proportion entre 15 et 65 % en poids et les composants de sérine c) en proportions entre 0,1 et 5,0 % en poids, chacun par rapport à la formulation totale.

5. Composition selon l'une des revendications 1 à 4, **caractérisée en ce qu'**elle contient en outre un acide gras oméga 3, en particulier, en quantités entre 30 et 80 % en poids par rapport à la formulation totale.

6. Composition selon la revendication 5, **caractérisée en ce qu'**elle contient en outre des composants d'acides gras d'acide docosahexaénique (DHA), d'acide α-linolénique (ALA) et d'acide eicosapentaénique (EPA), de préférence sous la forme de mono-, di- et/ou triglycérides et de mono-esters ou leurs mélanges.

7. Composition selon l'une des revendications 1 à 5, **caractérisée en ce qu'**elle contient d'autres composants, en particulier sous la forme de composants supplémentaires physiologiquement actifs ayant une action hyperémiante comme par exemple, le gingko biloba, une action neuroprotectrice et/ou anti-oxydante comme par exemple, la vitamine A, la vitamine C, la vitamine E, les polyphénols, le β-carotène, le sélénium, l'acide α-liponique, une action stimulant le métabolisme cérébral comme par exemple des complexes vitaminés (B₁, B₆, B₁₂, acide folique), les acides gras oméga 6, la créatine, la caféine et les glucides (glucose) et/ou des substances qui influent positivement sur la synthèse ou la libération de neurotransmetteurs comme par exemple, la choline et leurs sels (in)organiques, des donneurs d'acétyle comme par exemple, l'acétylcholine et/ou des substances qui influent positivement sur la biodisponibilité, la distribution et le métabolisme des phospholipides comme par exemple, les agonistes PPAR, les agonistes du récepteur de l'acide rétinique et des hypocholestérolémiants, et/ou d'autres composants sous la forme d'auxiliaires de formulation tels que des substances de remplissage, des anti-agglomérants, des arômes, des colorants.

8. Composition selon l'une des revendications 1 à 7, **caractérisée en ce qu'**elle se présente sous forme de formulation liquide, de préférence sous forme de capsules ou de poudre, de préférence, sous forme de comprimés ou de dragées.

9. Utilisation de la composition selon l'une des revendications 1 à 8, pour la production d'un agent destiné à améliorer et à renforcer la fonction cérébrale et la mémoire.

10. Utilisation de la composition selon l'une des revendications 1 à 8, comme complément alimentaire (dietary supplement), aliment fonctionnel (functional food) ou comme alimentation spéciale.

11. Utilisation selon l'une des revendications 9 ou 10, associée à une détresse corporelle ou mentale, en particulier dans le cadre d'activités sportives.
